(19) Europäisches Patentamt
European Patent Office
Office européen des brevets

(11) Veröffentlichungsnummer: **0 023 636**
**B1**

(12) # EUROPÄISCHE PATENTSCHRIFT

(45) Veröffentlichungstag der Patentschrift: **17.11.82**

(51) Int. Cl.³: **C 07 F 9/65,** A 01 N 57/16

(21) Anmeldenummer: **80104189.8**

(22) Anmeldetag: **17.07.80**

(54) **Pyrazolin-5-on-yl-methyl-(di)thiophosphorsäureester, Verfahren zu ihrer Herstellung und ihre Verwendung zur Bekämpfung von Schädlingen.**

(30) Priorität: **04.08.79 DE 2931673**

(43) Veröffentlichungstag der Anmeldung: **11.02.81 Patentblatt 81/6**

(45) Bekanntmachung des Hinweises auf die Patenterteilung: **17.11.82 Patentblatt 82/46**

(84) Benannte Vertragsstaaten: **AT BE CH DE FR GB IT LI NL SE**

(56) Entgegenhaltungen:
**FR-A-1 331 721**
**FR-A-2 100 109**
**HELVETIA CHIMICA ACTA, Band 56, Fasciculis 7, 7. November 1973, K. RUFENACHT: »Arbeiten über Phosphorsäure- und Thiophosphorsäureester mit einem heterocyclischen Substituenten. Thio- und Dithiophosphorsäureester von der Art des GS 13 005 mit einem analogen oder homologen hete- reocyclischen Ring«, Seiten 2186—2204.**

(73) Patentinhaber: **BASF Aktiengesellschaft, Carl-Bosch-Strasse 38, D-6700 Ludwigshafen (DE)**

(72) Erfinder: **Loeffler, Hans-Peter, Dr., Defreggerstrasse 14, D-6700 Ludwigshafen (DE)**
Erfinder: **Adolphi, Heinrich, Dr., Kalmitweg 11, D-6703 Limburgerhof (DE)**

EP 0 023 636 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Pyrazolin-5-on-yl-methyl-(di)thiophosphorsäureester, Verfahren zu ihrer Herstellung und ihre Verwendung zur Bekämpfung von Schädlingen

Die vorliegende Erfindung betrifft neue Pyrazolin-5-on-yl-methyl-(di)thiophosphorsäureester, ein Verfahren zu ihrer Herstellung und Schädlingsbekämpfungsmittel, die diese Phosphorsäureester als Wirkstoffe enthalten.

(3,4,4-Trimethyl-2-pyrazolin-5-on-yl)-methyl-(di)thiophosphorsäureester sind aus Helv. Chim. Acta, 56, 2188, 2189 (1973), bekannt, haben jedoch bisher keine Bedeutung als Schädlingsbekämpfungsmittel erlangt.

Es wurde nun gefunden, daß Pyrazolin-5-on-yl-methyl-(di)thiophosphorsäureester der Formel I

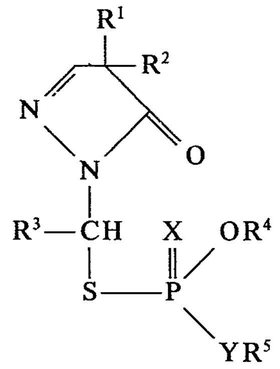

(I)

in der

$R^1$ und $R^2$ gleich oder verschieden sind und eine Alkylgruppe mit 1 bis 5 Kohlenstoffatomen, Allyl, Phenyl, $R^1$ zusätzlich Methoxycarbonylmethyl,

$R^3$ Wasserstoff oder eine gegebenenfalls durch Halogen substituierte Methylgruppe,

$R^4$ Methyl oder Äthyl,

$R^5$ eine Alkylgruppe mit 1 bis 5 Kohlenstoffatomen,

X Sauerstoff oder Schwefel und

Y Sauerstoff oder Schwefel bedeuten, wobei X und Y nicht gleichzeitig Schwefel sein können,

Schädlinge, insbesondere aus der Klasse der Insekten und Spinnentiere, wirksam bekämpfen und überraschenderweise den bekannten Verbindungen, die in 3-Stellung am Pyrazolinonring eine Methylgruppe tragen, in ihrer Wirkung überlegen sind.

In der Formel I sind $R^1$ und $R^2$ gleich oder verschieden und stehen für unverzweigte oder verzweigte Alkylgruppen mit 1 bis 5 Kohlenstoffatomen, wie Methyl, Äthyl, n-Propyl, Isopropyl, Isobutyl, sec.-Butyl, Isopentyl, insbesondere Methyl, für Allyl oder Phenyl. $R^3$ in Formel I steht für Wasserstoff oder eine gegebenenfalls halogensubstituierte Methylgruppe, wie Chlormethyl oder Brommethyl. $R^5$ in Formel I bedeutet eine unverzweigte oder verzweigte Alkylgruppe mit 1 bis 5 Kohlenstoffatomen, wie Methyl, Äthyl, n-Propyl, i-Propyl, sec.-Butyl, i-Butyl, 1-Methyl-n-butyl.

Bevorzugte Verbindungen der Formel I sind solche, bei denen $R^1$ und $R^2$ Methyl, $R^3$ Wasserstoff und $R^4$ und $R^5$ Alkyl mit 1 bis 4 Kohlenstoffatomen, bedeuten.

Die Pyrazolin-5-on-yl-methyl-(di)thiophosphorsäureester der Formel I können durch Umsetzung von Pyrazolinonen der Formel II

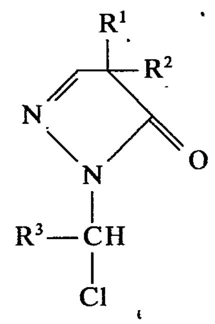

(II)

in der

$R^1$, $R^2$ und $R^3$ die obengenannten Bedeutungen haben, mit (Di)Thiophosphorsäuren der Formel III

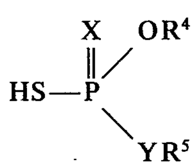

(III)

in der
$R^4$, $R^5$, X und Y die obengenannten Bedeutungen haben, in Gegenwart eines säurebindenden Mittels erhalten werden.

Ebenso kann auch ein Pyrazolinon der Formel II mit einem Salz einer (Di)Thiophosphorsäure der Formel III direkt umgesetzt werden, wobei auf den Zusatz eines säurebindenden Mittels verzichtet werden kann. Vorzugsweise verwendet man dabei Alkalimetallsalze, beispielsweise das Kaliumsalz, oder das Ammoniumsalz des Esters.

Beide Umsetzungen können in Gegenwart eines organischen Lösungs- oder Verdünnungsmittels, wie Aceton, Acetonitril oder Toluol, bei einer Temperatur im Bereich zwischen 20 und 100°C durchgeführt werden.

Geeignete säurebindende Mittel für die zuerst genannte Verfahrensvariante sind Basen, wie Alkali- und Erdalkalicarbonate, -hydroxide und -hydrogencarbonate.

Die Ausgangsstoffe der Formel II, bei denen $R^3$ Wasserstoff bedeutet, lassen sich durch Chlormethylierung aus Verbindungen der Formel IV

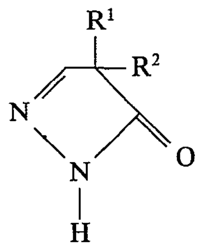

(IV)

in der $R^1$ und $R^2$ die obengenannten Bedeutungen haben, herstellen.

Dabei kann die Verbindung der Formel IV mit Paraformaldehyd und Thionylchlorid in einem Lösungsmittel, wie Dichlormethan oder Toluol, einstufig zum Pyrazolinon der Formel II umgesetzt oder aber mit Formaldehyd hydroxymethyliert werden, wonach die so hergestellte N-Hydroxy-methylverbindung dann mit Chlorierungsmitteln, wie Thionylchlorid oder Phosphorpentachlorid, in einem Lösungsmittel, beispielsweise Dichlormethan oder Toluol, in das Pyrazolinon der Formel II überführt wird.

Die Ausgangsstoffe der Formel II, bei denen $R^3$ Halogenmethyl bedeutet, lassen sich ebenfalls aus Verbindungen der Formel IV herstellen. Dabei wird ein Pyrazolinon der Formel IV in Anlehnung an bekannte Verfahren mit Vinylacetat gegebenenfalls in Anwesenheit von Katalysatoren, wie Quecksilbersalzen, zur N-Vinylverbindung umgesetzt, die sich zum Pyrazolinon der Formel II halogenieren läßt.

Die Verbindungen der Formel IV lassen sich in Anlehnung an bekannte Verfahren herstellen (Rec. Trav. Chim., 45, 82 ff [1926]).

Die (Di)Thiophosphorsäureester der Formel III sind bekannt und können nach bekannten Verfahren erhalten werden.

Die folgenden Beispiele erläutern die Herstellung der erfindungsgemäßen Verbindungen.

### Beispiel 1

Zu 19,5 g 2-Formyl-2-methyl-propionsäuremethylester in 300 ml Äthanol gibt man 7,5 g Hydrazinhydrat; die Lösung erwärmt sich dabei schwach. Danach wird noch 5 Stunden unter Rückfluß erhitzt. Die Lösung wird eingeengt, das zurückbleibende rohe Produkt wird aus Cyclohexan umkristallisiert. Man erhält 15 g 4,4-Dimethyl-2-pyrazolin-5-on vom Schmelzpunkt 97 bis 98° C.

Zu einer Suspension von 11,2 g des so hergestellten 4,4-Dimethyl-2-pyrazolin-5-on in 50 ml Toluol und 3,3 g Para-formaldehyd tropft man unter Rühren 13,1 g Thionylchlorid. Die entstehende Lösung läßt man zwei Tage lang bei 20°C stehen und entfernt danach unter vermindertem Druck das Lösungsmittel. Der Rückstand (14,8 g) ist ein Öl vom Brechungsindex $n_D^{24}$ 1.4870, das im wesentlichen aus 1-Chlormethyl-4,4-dimethyl-2-pyrazolin-5-on besteht und ohne zusätzliche Reinigung weiter umgesetzt werden kann.

Zu 12,9 g 1-Chlormethyl-4,4-dimethyl-2-pyrazolin-5-on in 100 ml Acetonitril gibt man in mehreren Portionen 14 g Ammonium-O,O-dimethyldithiophosphat. Unter Temperaturanstieg von 20 auf 40°C beginnt Ammoniumchlorid auszufallen. Man rührt 4 Stunden bei Raumtemperatur nach und destilliert das Lösungsmittel unter vermindertem Druck ab. Der Rückstand wird mit 300 ml Toluol versetzt und mit Wasser gewaschen. Die organische Phase wird abgetrennt und über Natriumsulfat getrocknet. Nach Abziehen des Lösungsmittels wird der Rückstand mit Petroläther versetzt, die entstandenen Kristalle werden abgesaugt und mit Petroläther gewaschen. Man erhält 15,3 g Dithiophosphorsäure-O,O-dimethyl-S-(4,4-dimethyl-2-pyrazolin-5-on-yl-methyl)-ester vom Schmelzpunkt 75 bis 76° C.

Beispiel 2

Zu 23,4 g 2-Methyl-2-formyl-penten-4-carbonsäuremethylester in 300 ml Äthanol gibt man 7,5 g Hydrazinhydrat. Man erhitzt 8 Stunden unter Rückfluß, engt die Lösung ein und kristallisiert den Rückstand aus Cyclohexan um. Man erhält 4-Methyl-3-allyl-2-pyrazolin-5-on vom Schmelzpunkt 47° C.

Zu 13,8 g 4-Methyl-4-allyl-2-pyrazolin-5-on, gelöst in 50 ml Toluol, gibt man 3,3 g Paraformaldehyd und 13,1 g Thionylchlorid. Man läßt 2 Tage lang stehen und entfernt das Lösungsmittel unter vermindertem Druck. Es verbleiben 15 g 1-Chlormethyl-4-methyl-4-allyl-2-pyrazolin-5-on als orangefarbenes Öl.

5,95 g 1-Chlormethyl-4-methyl-4-allyl-2-pyrazolin-5-on werden mit 12,29 g Ammonium-O,O-diäthylmonothiophosphat in 50 ml Acetonitril 5 Stunden bei 20° C und 1 Stunde bei 50° C gerührt. Dann wird unter vermindertem Druck eingeengt, der Rückstand wird mit Toluol versetzt und mit Wasser gewaschen. Die organische Phase wird abgetrennt und über Natriumsulfat getrocknet. Nach Entfernen des Lösungsmittels unter vermindertem Druck erhält man 8,5 g Thiophosphorsäure-O,O-diäthyl-S-(4-methyl-4-allyl-2-pyrazolin-5-on-yl-methyl)-ester; $n_D^{23}$ =1.4962.

Analog lassen sich beispielsweise folgende Verbindungen der Formel I herstellen:

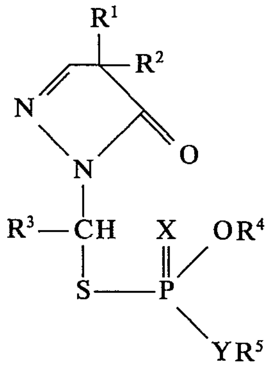

| Nr. | $R^1$ | $R^2$ | $R^3$ | X | $R^4$ | $YR^5$ | Physik. Daten |
|---|---|---|---|---|---|---|---|
| 1 | $CH_3$ | $CH_3$ | H | O | $CH_3$ | $OCH_3$ | $n_D^{22}$ 1.5011 |
| 2 | $CH_3$ | $CH_3$ | H | O | $C_2H_5$ | $OC_2H_5$ | $n_D^{22}$ 1.4941 |
| 3 | $CH_3$ | $CH_3$ | H | S | $CH_3$ | $OCH_3$ | Fp. 75–76°C |
| 4 | $CH_3$ | $CH_3$ | H | S | $C_2H_5$ | $OC_2H_5$ | $n_D^{31}$ 1.5220 |
| 5 | $CH_3$ | $CH_3$ | $CH_2Cl$ | O | $CH_3$ | $OCH_3$ | |
| 6 | $CH_3$ | $CH_3$ | $CH_2Cl$ | S | $CH_3$ | $OCH_3$ | |
| 7 | $CH_3$ | $CH_3$ | H | O | $C_2H_5$ | $S—n-C_3H_7$ | $n_D^{22}$ 1.5272 |
| 8 | $CH_3$ | $CH_3$ | H | O | $C_2H_5$ | $S—sec-C_4H_9$ | |
| 9 | $C_6H_5$ | $CH_3$ | H | S | $CH_3$ | $OCH_3$ | |
| 10 | $C_6H_5$ | $CH_3$ | H | O | $CH_3$ | $OCH_3$ | |
| 11 | $C_6H_5$ | $CH_3$ | H | S | $C_2H_5$ | $OC_2H_5$ | |
| 12 | $C_6H_5$ | $CH_3$ | H | O | $C_2H_5$ | $OC_2H_5$ | |
| 13 | $CH_2{=}CH—CH_2$ | $CH_3$ | H | O | $CH_3$ | $OCH_3$ | $n_D^{25}$ 1.5081 |
| 14 | $CH_2{=}CH—CH_2$ | $CH_3$ | H | O | $C_2H_5$ | $OC_2H_5$ | $n_D^{23}$ 1.4962 |
| 15 | $CH_2{=}CH—CH_2$ | $CH_3$ | H | S | $CH_3$ | $OCH_3$ | $n_D^{24}$ 1.5361 |
| 16 | $CH_2{=}CH—CH_2$ | $CH_3$ | H | S | $C_2H_5$ | $OC_2H_5$ | $n_D^{24}$ 1.5310 |
| 17 | $CH_2COOCH_3$ | $CH_3$ | H | O | $CH_3$ | $OCH_3$ | |
| 18 | $CH_2COOCH_3$ | $CH_3$ | H | S | $CH_3$ | $OCH_3$ | |

Die erfindungsgemäßen Pyrazolin-5-on-methyl-(di)thiophosphorsäureester der Formel I sind geeignet, Schädlinge aus der Klasse der Insekten und Spinnentiere (Arachnoidea) wirksam zu bekämpfen. Sie können im Pflanzenschutz sowie auf dem Hygiene-, Vorratsschutz- und Veterinärsektor eingesetzt werden.

Zu diesen Schädlingen gehören Insekten aus der Ordnung der Schmetterlinge (Lepidoptera), beispielsweise Plutella maculipennis (Kohlschabe), Leucoptera coffeèlla (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana

(Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earis insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);
aus der Ordnung der Käfer (Coleoptera), beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aenus (Rapsglanzkäfer), Atomaria linearis (Moosknopfkäfer), Epilachna varivestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);
aus der Ordnung der Zweiflügler (Diptera), beispielsweise Lycoria pectoralis, Mayetiola destructor (Hessenfliege), Dasineura brassicae (Kohlschoten-Gallmücke), Contarinia tritici (Gelbe Weizen-Gallmücke), Contarinia nasturtii (Drehherzmücke), Contarinia pyrivora (Birnen-Gallmücke), Haplodiplosis equestris (Sattelmücke), Tipula paludosa (Wiesenschnake), Tipula oleracea (Kohlschnake), Anopheles maculipennis, Culex pipiens, Aedes aegypti (Gelbfiebermücke), Aedes vexans, Tabanus bovinus (Rinderbremse), Merodon equestris (Große Narzissenfliege), Dacus cucurbitae (Melonenfliege), Dacus oleae (Olivenfliege), Ceratitis capitata (Mittelmeer-Fruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Psila rosae (Möhrenfliege), Musca domestica (Stubenfliege);
aus der Ordnung der Hautflügler (Hymenoptera), beispielsweise Athalia rosae (Rübenblattwespe), Hoplocampa minuta (Pflaumensägewespe), Monomorium pharaonis (Pharaoameise), Solenopsis germinata (Feuerameise), Atta sexdens (Blattschneiderameise), Vespa crabro (Hornisse);
aus der Ordnung der Schnabelkerfen (Rhynchota), beispielsweise Aelia acuminata (Getreidespitzwanze), Nezara viridula (Grüne Reiswanze), Antestia faceta (Braune Kaffeewanze), Eurygaster integriceps (Getreidewanze), Eurygaster maura (Breitbauchwanze), Anasa tristis (Squash bug), Blissus leucopterus (Chinch bug), Dysdercus cingulatus (Kapok-Wanze), Dysdercus intermedius (Baumwollwanze), Piesma quadrata (Rübenwanze), Lygus hesperus (Tarnished plant bug), Lygus pratensis (Wiesenwanze), Sahlbergella singularis (Kakaorindenwanze), Helopeltis antonii (Teewanze), Cimex lectularius (Bettwanze), Triatoma megista, Rhodnius prolixus, Perkinsiella saccharicida (Zuckerrohrzikade), Peregrinus maidis (Corn leafhopper), Nilapervata lugens (Braune Zikade), Nilapervata cryzae, Tomaspis saccharina (Sugar cane froghopper), Iocerus clypealis (Mangohopper), Typhlocyba frogathi (Apple leaf Jassid), Empoasca fabae Kartoffelzikade), Empoasca lybica, Circulifer tenellus (Beet leafhopper), Nephotettix bipunctatus (Green rice liefhopper), Macrosteles laevis, Dalbulus maidis (Corn leafhopper), Psylla mali (Apfelblattsauger), Psylla piri (Birnblattsauger), Trialeurodes vaporariorum (Weiße Fliege), Bemisia tabaci (Cotton white-fly), Hyalopterus pruni (Mehlige Pflaumenblattlaus), Aphis fabae (Schwarze Bohnenlaus), Aphis pomi (Grüne Apfellaus), Rhopalasiphon maidis (Maisblattlaus), Aphidula nasturtii (Kreuzdornblattlaus), Cerosipha gossypii (Gurkenblattlaus), Toxoptera aurantii (Black orange aphid), Sappaphis mali (Rosige Apfellaus), Sappaphis mala (Mehlige Birnenblattlaus), Dysaphis radicola (Mehlige Apfelfaltenlaus), Brachycaudus helichrysi (Kleine Pflaumenlaus), Brachycaudus cardui (Große Pflaumenlaus), Brevicoryne brassicae (Kohlblattlaus), Phorodon humuli (Hopfenblattlaus), Rhopalomyzus ascalonicus (Zwiebellaus), Myzus persicae (Pfirsichblattlaus), Myzus cerasi (Schwarze Sauerkirschenlaus), Crypromyzus ribis (Johannisbeerblasenlaus), Dysaulacorthum pseudosolani (Gefleckte Kartoffellaus), Acyrthosiphon onobrychis (Grüne

Erbsenlaus), Macrosiphon rosae (Große Rosenblattlaus), Macrosiphon solani (Gestreifte Kartoffellaus), Megoura viciae (Wickenlaus), Schizoneura lanuginosa (Birnenblutlaus), Eriosoma lanigerum (Blutlaus), Pemphigius bursarius (Salatwurzellaus), Dreyfusia nordmannianae (Tannentrieblaus), Adelges laricis (Rote Fichtengallenlaus), Sacchiphantes abietis (Gelbe Fichtengallenlaus), Vitcus vitifolii (Reblaus), Icerya purchasi (Australische Wollschildlaus), Pseudococcus aonidum (Longtailed mealybug), Planococcus citri (Citrus-Schmierlaus), Coccus hesperidum (Soft scale), Eulecanium corni (Napfschildlaus), Saissetia coffeae, Aspidiotus perniciosus (San Josè-Schildlaus), Aonidiella aurantii (Red scale);

aus der Ordnung der Geradflügler (Orthoptera), beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe);

aus der Ordnung der Termiten (Isoptera), beispielsweise Calotermes flavicollis, Leucotermes flavipes, Reticulitermes lucifugus, Termes natalensis;

aus der Ordnung der Fransenflügler (Thysanoptera), beispielsweise Heliothrips fasciatus, Heliothrips femoralis, Heiolthrips haemorrhoidalis, Selenothrips rubrocinctus, Frankiniella fusca, Frankiniella tritici, Kakothrips robustus, Thrips tabaci;

aus der Ordnung der Tierläuse (Phthiraptera), beispielsweise Pediculus humanus (Menschenlaus), und aus der Ordnung der Flöhe (Siphonaptera), beispielsweise Pulex irritans (Menschenfloh), Sarcopsylla penetrans (Sandfloh), Xenopsylla cheopis (Rattenfloh).

Zur Klasse der Spinnentiere (Arachnoidea) gehören Milben und Zecken (Acarina), beispielsweise Ixodes ricinus (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa. Die Wirkstoffe können als solche, in Form ihrer Formulierungen oder den daraus bereiteten Anwendungsmöglichkeiten, z. B. in Form von direkt versprübaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenon, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 3 Gewichtsteile Dithiophosphorsäure-O,O-dimethyl-S-[4,4-dimethyl-2-pyrazolin-5-on-yl-methyl]-ester werden mit 97 Gewichtsteilen feinteiligem Kaolin innig vermischt. Man erhält auf diese Weise ein Stäubemittel, das 3 Gew.-% des Wirkstoffs enthält.

II. 30 Gewichtsteile Thiophosphorsäure-O,O-dimethyl-S-[4,4-dimethyl-2-pyrazolin-5-on-yl-methyl]-ester werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfestigkeit.

III. 20 Gewichtsteile Dithiophosphorsäure-O-äthyl-S-n-propyl-S-[4,4-dimethyl-2-pyrazolin-5-on-yl-methyl]-ester werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

IV. 20 Gewichtsteile Thiophosphorsäure-O,O-dimethyl-S-[4-methyl-4-allyl-2-pyrazolin-5-on-yl-methyl]-ester werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%. Die Wirkstoffe können in Form dieser Formulierungen oder aber in Form daraus herstellbarer anwendungsfertiger Zubereitungen eingesetzt werden. Die Wirkstoffkonzentrationen in den anwendungsfertigen Zubereitungen können in weiten Bereichen variiert werden. Im allgemeinen liegen sie zwischen 0,0001 und 10%, vorzugsweise zwischen 0,01 und 10%.

Die Wirkstoffe können auch mit gutem Erfolg im Ultra-Low-Volume-Verfahren (ULV) verwendet werden. Dabei ist es möglich, Formulierungen mit über 95 Gew.-% Wirkstoff oder sogar den Wirkstoff ohne Zusätze auszubringen.

Zu den Wirkstoffen können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden:

1,2-Dibrom-3-chlorpropan,
1,3-Dichlorpropen,
1,3-Dichlorpropen + 1,2-Dichlorpropan,
1,2-Dibrom-äthan,
2-sec.-Butylphenyl-N-methylcarbamat,
o-Chlorphenyl-N-methylcarbamat,
3-Isopropyl-5-methylphenyl-N-methylcarbamat,
o-Isopropoxyphenyl-N-methylcarbamat,
3,5-Dimethyl-4-methylmercapto-phenyl-N-methylcarbamat,
4-Dimethylamino-3,5-xylyl-N-methylcarbamat,
2-(1,3-Dioxolan-2-yl)-phenyl-N-methylcarbamat,
1-Naphthal-N-methylcarbamat,
2,3-Dihydro-2,2-dimethyl-benzofuran-7-yl-N-methylcarbamat,
2,2-Dimethyl-1,3-benzodioxol-4-yl-N-methylcarbamat,
2-Dimethylamino-5,6-dimethyl-4-pyrimidinyl-dimethylcarbamat,
2-Methyl-2-(methylthio)-propionaldehyd-O-(methylcarbamoyl)-oxim,
S-Methyl-N-[(methylcarbamoyl)-oxy]-thio-acetimidat,
Methyl-N',N'-dimethyl-N-[(methylcarbamoyl)oxy]-1-thiooxamidat,
N-(2-Methyl-4-chlor-phenyl)-N',N'-dimethylformamidin,
Tetrachlorthiophen, 1-(2,6-Difluor-benzoyl)-3-(4-chlor-phenyl)-harnstoff,
O,O-Dimethyl-O-(p-nitrophenyl)-phosphorthioat,

O,O-Diäthyl-O-(p-nitrophenyl)-phosphorthioat,
O-Äthyl-O-(p-nitrophenyl)-phenyl-phosphonothioat,
O,O-Dimethyl-O-(3-methyl-4-nitrophenyl)-phosphorthioat,
O,O-Diäthyl-O-(2,4-dichlorphenyl)-phosphorthioat,
O-Äthyl-O-(2,4-dichlorphenyl)-phenyl-phosphonothioat,
O,O-Dimethyl-O-(2,4,5-trichlorphenyl)-phosphorthioat,
O-Äthyl-O-(2,4,5-trichlorphenyl)-äthyl-phosphonothioat,
O,O-Dimethyl-O-(4-brom-2,5-dichlorphenyl)-phosphorthioat,
O,O-Dimethyl-O-(2,5-dichlor-4-jodphenyl)-phosphorthioat,
O,O-Dimethyl-O-(3-methyl-4-methylthiophenyl)-phosphorthioat,
O-Äthyl-O-(3-methyl-4-methylthiophenyl)-isopropyl-phosphoramidat,
O,O-Diäthyl-O-[p-(methylsulfinyl)phenyl]-phosphorthioat,
O-Äthyl-S-phenyl-äthyl-phosphonodithioat,
O,O-Diäthyl-[2-chlor-1-(2,4-dichlorphenyl)-vinyl]-phosphat,
O,O-Dimethyl-[2-chlor-1-(2,4,5-trichlorphenyl)]-vinyl-phosphat,
O,O-Dimethyl-S-(1′-phenyl)-äthylacetat-phosphordithioat,
Bis-(dimethylamino)-fluorphosphinoxid,
Octamethyl-pyrophosphoramid,
O,O,O,O-Tetraäthyldithio-pyrophosphat,
S-Chlormethyl-O,O-diäthyl-phosphordithioat,
O-Äthyl-S,S-dipropyl-phosphordithioat,
O,O-Dimethyl-O-2,2-dichlorvinyl-phosphat,
O,O-Dimethyl-1,2-dibrom-2,2-dichloräthylphosphar,
O,O-Dimethyl-2,2,2-trichlor-1-hydroxy-äthylphosphonat,
O,O-Dimethyl-S-[1,2-biscarbäthoxy-äthyl-(1)]-phosphordithioat,
O,O-Dimethyl-O-(1-methyl-2-carbmethoxy-vinyl)-phosphat,
O,O-Dimethyl-S-(N-methyl-carbamoyl-methyl)-phosphordithioat,
O,O-Dimethyl-S-(N-methyl-carbamoyl-methyl)-phosphorthioat,
O,O-Dimethyl-S-(N-methoxyäthyl-carbamoyl-methyl)-phosphordithioat,
O,O-Dimethyl-S-(N-formyl-N-methyl-carbamoylmethyl-phosphordithioat,
O,O-Dimethyl-O-[1-methyl-2-(methyl-carbamoyl)-vinyl]-phosphat,
O,O-Dimethyl-O-[(1-methyl-2-dimethylcarbamoyl)-vinyl]-phosphat,
O,O-Dimethyl-O-[(1-methyl-2-chlor-2-diäthylcarbamoyl)-vinyl]-phosphat,
O,O-Diäthyl-S-(äthylthio-methyl)-phosphordithioat,
O,O-Diäthyl-S-[(p-chlorphenylthio)-methyl]-phosphordithioat,
O,O-Dimethyl-S-(2-äthylthioäthyl)-phosphorthioat,
O,O-Dimethyl-S-(2-äthylthioäthyl)-phosphordithioat,
O,O-Dimethyl-S-(2-äthylsulfinyl-äthyl)-phosphorthioat,
O,O-Diäthyl-S-(2-äthylthio-äthyl)-phosphordithioat,
O,O-Diäthyl-S-(2-äthylsulfinyl-äthyl)-phosphorthioat,
O,O-Diäthyl-thiophosphoryliminophenyl-acetonitril,
O,O-Diäthyl-S-(2-chlor-1-phthalimidoäthyl)-phosphordithioat,
O,O-Diäthyl-S-[6-chlor-benzoxazolon-(2)-yl(3)]-methyldithiophosphat,
O,O-Dimethyl-S-[2-methoxy-1,3,4-thiadiazol-5-[4H]-onyl-(4)-methyl]-phosphordithioat,
O,O-Diäthyl-O-[3,5,6-trichlor-pyridyl-(2)]-phosphorthioat,
O,O-Diäthyl-O-(2-pyrazinyl)-phosphorthioat,
O,O-Diäthyl-O-[2-isopropyl-4-methyl-pyrimidinyl(6)]-phosphorthioat,
O,O-Diäthyl-O-[2-(diäthylamino)-6-methyl-4-pyrimidinyl]-thionophosphat,
O,O-Dimethyl-S-(4-oxo-1,2,3-benzotriazin-3-[4H]-yl-methyl)-phosphordithioat,
O,O-Dimethyl-S-[(4,6-diamino-1,3,5-triazin-2-yl)-methyl]-phosphordithioat,
O,O-Diäthyl-(1-phenyl-1,2,4-triazol-3-yl)-thionophosphat,
O,S-Dimethyl-phosphor-amido-thioat,
O,S-Dimethyl-N-acetyl-phosphoramidothioat,
γ-Hexachlorcyclohexan,
1,1-Di-(p-methoxyphenyl)-2,2,2-trichlor-äthan,
6,7,8,9,10,10-Hexachloro-1,5,5a,6,9,9a-hexahydro-6,9-methano-2,4,3-benzodioxathiepin-3-oxid,
Pyrethrine,
DL-2-Allyl-3-methyl-cyclopenten-(2)-on-(1)-yl-(4)-DL-cis,trans-chrysanthemat,
5-Benzyl-furyl-(3)-methyl-DL-cis,trans-chrysanthemat,
3-Phenoxybenzyl(±)-cis,trans-2,2-dimethyl-3-(2,2-dichlorvinyl)-cyclopropancarboxylat,
α-Cyano-3-phenoxybenzyl(±)-cis,trans-2,2-dimethyl-3-(2,2-dichlorvinyl)-cyclopropancarboxylat,
(s)-α-Cyano-3-phenoxybenzyl-cis(1R,3R)-2,2-dimethyl-3-(2,2-dibromvinyl)-cyclopropan-

carboxylat,
3,4,5,6-Tetrahydrophthalimidoäthyl-DL-cis,trans-chrysanthemat,
2-Methyl-5-(2-propinyl)-3-furylmethyl-chrysanthemat,
$\alpha$-Cyano-3-phenoxybenzyl-$\alpha$-isopropyl-4-chiorphenylacetat.

Die folgenden Beispiele belegen die biologische Wirkung der neuen Verbindungen. Vergleichsmittel sind die aus Helv. Chim. Acta, 56, 2189 (1973), bekannten Verbindungen

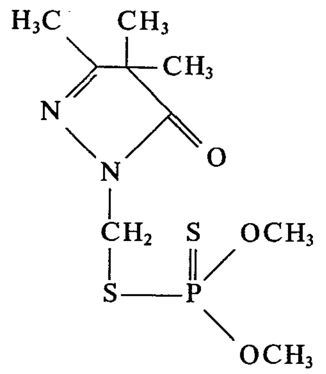

(I)

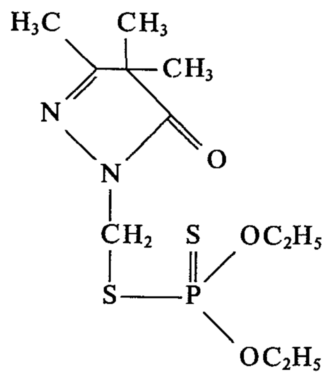

(II)

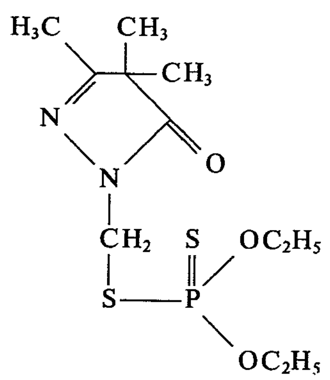

(III)

Die Numerierung der erfindungsgemäßen Wirkstoffe entspricht der tabellarischen Auflistung.

### Beispiel A

### Kontaktwirkung auf Stubenfliegen (Musca domestica); Dauerkontakt

Beide Teile einer Petrischale von 10 cm Durchmesser werden mit insgesamt 2 ml der acetonischen Wirkstofflösung ausgekleidet. Nach Verdunsten des Lösungsmittels (ca. 30 Minuten) bringt man je 10 Fliegen in die Schalen. Die Mortalitätsrate wird nach 4 Stunden festgestellt.

| Wirkstoff Nr. | Wirkstoffmenge [mg/Petrischale] | Mortalitätsrate [%] |
|---|---|---|
| 1 | 2,0 | 100 |
| | 0,2 | 100 |
| 4 | 2,0 | 100 |
| 13 | 0,2 | 80 |
| | 2,0 | |
| | 0,2 | 100 |
| | | 80 |
| 14 | 2,0 | 100 |
| | 0,2 | 80 |
| 15 | 2,0 | 100 |
| | 0,2 | 80 |
| 16 | 2,0 | 100 |
| | 0,2 | 100 |
| I | 2,0 | 100 |
| | 0,2 | <30 |
| II | 2,0 | 100 |
| | 0,2 | <30 |
| III | 2,0 | 100 |
| | 0,2 | <30 |

Beispiel B

Kontaktwirkung auf Baumwollwanzen (Dysdercus intermedius)

Petrischalen von 10 cm Durchmesser werden mit 1 ml der acetonischen Wirkstofflösung ausgekleidet. Nach dem Verdunsten des Lösungsmittels besetzt man die Schalen mit je 20 Larven des vorletzten Stadiums und registriert die Wirkung nach 24 Stunden.

| Wirkstoff Nr. | Wirkstoffmenge pro Petrischale [mg] | Mortalitätsrate [%] |
|---|---|---|
| 1 | 0,002 | 80 |
| 2 | 0,02 | 100 |
| 3 | 0,002 | 100 |
| 4 | 0,01 | 80 |
| 13 | 0,02 | 100 |
| 15 | 0,02 | 80 |

## Beispiel C

### Kontaktwirkung auf Blattläuse (Aphis fabae); Spritzversuch

Getopfte Bohnenpflanzen (Vicia faba) mit starken Blattlauskolonien werden in der Spritzkammer mit den wäßrigen Wirkstoffaufbereitungen tropfnaß gespritzt. Die Auswertung erfolgt nach 24 Stunden.

| Wirkstoff Nr. | Wirkstoffkonzentration der wäßrigen Aufbereitung [Gew.-%] | Mortalitätsrate [%] |
|---|---|---|
| 1 | 0,01 | 100 |
| 3 | 0,005 | ca. 80 |
| 4 | 0,02 | 100 |
| 13 | 0,01 | 100 |
| 14 | 0,01 | 100 |

## Beispiel D

### Systemische Wirkung auf Blattläuse (Aphis fabae); Gießversuch

Bohnenpflanzen, die in Plastiktöpfen von 8 cm Durchmesser, gefüllt mit 300 g Komposterde, stehen und stark mit Blattläusen (Aphis fabae) infiziert sind, werden mit 20 ml der wäßrigen Aufbereitung der Wirkstoffe angegossen.

Die Mortalitätsrate wird nach 48 Stunden ermittelt.

| Wirkstoff Nr. | Wirkstoffkonzentration der wäßrigen Aufbereitung [Gew.-%] | Mortalitätsrate [%] |
|---|---|---|
| 1 | 0,05 | ca. 80 |
| 3 | 0,01 | ca. 80 |
| 4 | 0,05 | 100 |
| 13 | 0,05 | 100 |
| 14 | 0,05 | 100 |
| I | 0,1 | unwirksam |
| II | 0,1 | unwirksam |
| III | 0,1 | unwirksam |

## Beispiel E

### Kontaktwirkung auf Zecken (Ornithodorus moubata)

Geprüft wird mit Zecken im 3. Larvenstadium. Dazu taucht man die Tiere, die sich in einem Papierbeutel befinden, für 3 Sekunden in die Prüfemulsion. Die Beutel werden dann frei aufgehängt. Nach 48 Stunden wird die Wirkung auf die Zecken beurteilt.

| Wirkstoff Nr. | Wirkstoffkonzentration der wäßrigen Aufbereitung [Gew.-%] | Mortalitätsrate [%] |
|---|---|---|
| 1 | 0,001 | 100 |
| 2 | 0,002 | 100 |
| 13 | 0,0005 | ca. 80 |
| 14 | 0,002 | 100 |
| 15 | 0,002 | 100 |
| 16 | 0,01 | 100 |
| I | 0,1 | unwirksam |
| II | 0,1 | unwirksam |
| III | 0,1 | unwirksam |

**Patentansprüche**
**für die benannten Vertragstaaten: BE, CH, DE, FR, GB, IT, LI, LU, NL, SE**

1. Pyrazolin-5-on-yl-methyl-(di)thiophosphorsäureester der Formel I

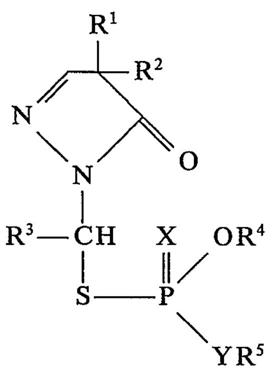

(I)

in der
$R^1$ und $R^2$ gleich oder verschieden sind und eine Alkylgruppe mit 1 bis 5 Kohlenstoffatomen, Allyl, Phenyl, $R^1$ zusätzlich Methoxycarbonylmethyl,
$R^3$ Wasserstoff oder eine gegebenenfalls durch Halogen substituierte Methylgruppe,
$R^4$ Methyl oder Äthyl,
$R^5$ eine Alkylgruppe mit 1 bis 5 Kohlenstoffatomen,
X Sauerstoff oder Schwefel und
Y Sauerstoff oder Schwefel bedeuten,
wobei X und Y nicht gleichzeitig Schwefel sein können.

2. Pyrazolin-5-on-yl-methyl-(di)thiophosphorsäureester der Formel I gemäß Anspruch 1, dadurch gekennzeichnet, daß $R^1$ und $R^2$ Methyl, $R^3$ Wasserstoff, $R^4$ Methyl oder Äthyl und $R^5$ Alkyl mit 1 bis 4 Kohlenstoffatomen bedeuten.

3. Verfahren zur Herstellung von Phosphorsäureestern der Formel I gemäß Anspruch 1, dadurch gekennzeichnet, daß man Pyrazolinone der Formel II

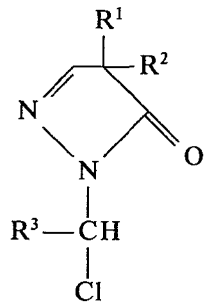

(II)

in der
$R^1$, $R^2$ und $R^3$ die in Anspruch1 angegebenen Bedeutungen haben, mit (Di)Thiophosphorsäuren der Formel III

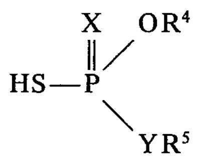

(III)

in der
$R^4$, $R^5$, X und Y die in Anspruch 1 angegebenen Bedeutungen haben, in Gegenwart eines säurebindenden Mittels oder mit Salzen der (Di)Thiophosphorsäuren der Formel III gegebenenfalls in Gegenwart eines organischen Lösungs- oder Verdünnungsmittels bei einer Temperatur im Bereich zwischen 20 und 100°C umsetzt.

4. Schädlingsbekämpfungsmittel, enthaltend einen Pyrazolin-5-on-yl-methyl-(di)thiophosphorsäureester der Formel I gemäß Anspruch 1.

5. Schädlingsbekämpfungsmittel, enthaltend einen festen oder flüssigen Trägerstoff und mindestens einen Pyrazolin-5-on-yl-methyl-(di)thiophosphorsäureester der Formel I gemäß Anspruch 1.

6. Insektizides und akarizides Mittel, enthaltend einen Pyrazolin-5-on-yl-methyl-(di)thiophosphorsäureester der Formel I gemäß Anspruch 1.

7. Verfahren zur Bekämpfung von Schädlingen, dadurch gekennzeichnet, daß man einen Pyrazolin-5-on-yl-methyl-(di)thiophosphorsäureester der Formel I gemäß Anspruch 1 auf die Schädlinge bzw. deren Lebensräume einwirken läßt.

**Patentansprüche für den benannten Vertragstaat: AT**

1. Schädlingsbekämpfungsmittel, enthaltend einen Pyrazolin-5-on-yl-methyl-(di)thiophosphorsäureester der Formel I

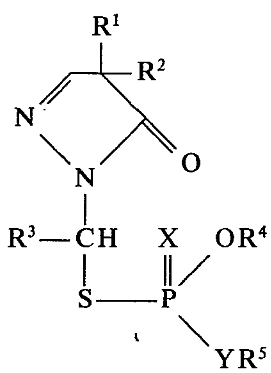

(I)

in der
$R^1$ und $R^2$ gleich oder verschieden sind und eine Alkylgruppe mit 1 bis 5 Kohlenstoffatomen, Allyl, Phenyl, $R^1$ zusätzlich Methoxycarbonylmethyl,
$R^3$ Wasserstoff oder eine gegebenenfalls durch Halogen substituierte Methylgruppe,
$R^4$ Methyl oder Äthyl,
$R^5$ eine Alkylgruppe mit 1 bis 5 Kohlenstoffatomen,
X Sauerstoff oder Schwefel und

Y Sauerstoff oder Schwefel bedeuten,
wobei X und Y nicht gleichzeitig Schwefel sein können.

2. Schädlingsbekämpfungsmittel, enthaltend einen Pyrazolin-5-on-yl-methyl-(di)thiophosphorsäureester der Formel I gemäß Anspruch 1, dadurch gekennzeichnet, daß $R^1$ und $R^2$ Methyl, $R^3$ Wasserstoff, $R^4$ Methyl oder Äthyl und $R^5$ Alkyl mit 1 bis 4 Kohlenstoffatomen bedeuten.

3. Verfahren zur Herstellung von Phosphorsäureestern der Formel I gemäß Anspruch 1, dadurch gekennzeichnet, daß man Pyrazolinone der Formel II

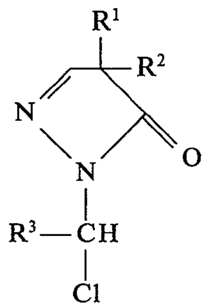

(II)

in der
$R^1$, $R^2$ und $R^3$ die in Anspruch 1 angegebenen Bedeutungen haben, mit (Di)Thiophosphorsäuren der Formel III

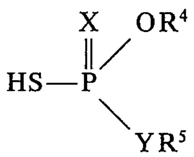

(III)

in der
$R^4$, $R^5$, X und Y die in Anspruch 1 angegebenen Bedeutungen haben, in Gegenwart eines säurebindenden Mittels oder mit Salzen der (Di)Thiophosphorsäuren der Formel III gegebenenfalls in Gegenwart eines organischen Lösungs- oder Verdünnungsmittels bei einer Temperatur im Bereich zwischen 20 und 100° C umsetzt.

4. Verfahren zur Bekämpfung von Schädlingen, dadurch gekennzeichnet, daß man einen Pyrazolin-5-on-yl-methyl-(di)thiophosphorsäureester der Formel I gemäß Anspruch 1 auf die Schädlinge bzw. deren Lebensräume einwirken läßt.

**Claims for the contracting States: BE, CH, DE, FR, GB, IT, LI, NL, SE**

1. Pyrazolin-5-onyl-methyl-(di)thiophosphoric acid esters of the formula I

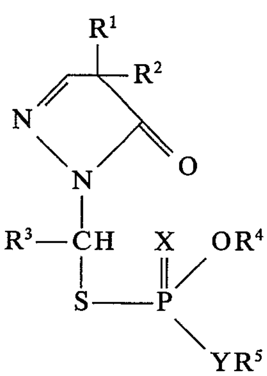

(I)

where
$R^1$ and $R^2$ are identical or different and denote an alkyl radical with 1 to 5 carbon atoms, allyl or phenyl,
$R^1$ additionally denotes methoxycarbonylmethyl,
$R^3$ denotes hydrogen or a methyl group which is optionally substituted by halogen,
$R^4$ denotes methyl or ethyl,
$R^5$ denotes an alkyl group with 1 to 5 carbon atoms,
X denotes oxygen or sulfur and
Y denotes oxygen or sulfur, but X and Y cannot both be sulfur at the same time.

2. Pyrazolin-5-onyl-methyl-(di)thiophosphoric acid esters of the formula I according to claim 1, characterized in that $R^1$ and $R^2$ denote methyl, $R^3$ denotes hydrogen, $R^4$ denotes methyl or ethyl and $R^5$ denotes alkyl with 1 to 4 carbon atoms.

3. Process for the preparation of phosphoric acid esters of the formula I according to claim 1, characterized in that pyrazolinones of the formula II

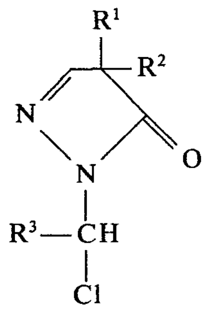

(II)

where $R^1$, $R^2$ and $R^3$ have the meanings given in claim 1, are reacted with (di)thiophosphoric acids of the formula III

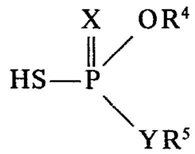

(III)

where $R^4$, $R^5$, X and Y have the meanings given in claim 1, in the presence of an acid acceptor, or with salts of the (di)thiophosphoric acids of the formula III, if appropriate in the presente of an organic solvent or diluent, at a temperature in the range of from 20 to 100° C.

4. Pest control agent containing a pyrazolin-5-onyl-methyl-(di)thiophosphoric acid ester of the formula I according to claim 1.

5. Pest control agent containing a solid or liquid carrier and one or more pyrazolin-5-onyl-methyl-(di)thiophosphoric acid esters of the formula I according to claim 1.

6. Insecticidal and acaricidal agent containing a pyrazolin-5-onyl-methyl-(di)thiophosphoric acid ester of the formula I according to claim 1.

7. Process for pest control, characterized in that a pyrazolin-5-onyl-methyl-(di)thiophosphoric acid ester of the formula I according to claim 1 is allowed to act on the pests or on their habitats.

**Claims for the contracting State: AT**

1. Pyrazolin-5-onyl-methyl-(di)thiophosphoric acid esters of the formula I

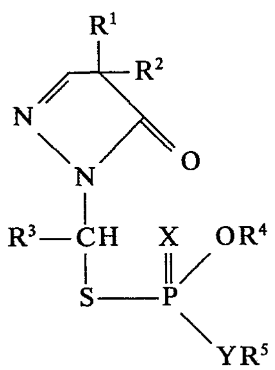

(I)

where
$R^1$ and $R^2$ are identical or different and denote an alkyl radical with 1 to 5 carbon atoms, allyl or phenyl,
$R^1$ additionally denotes methoxycarbonylmethyl,
$R^3$ denotes hydrogen or a methyl group which is optionally substituted by halogen,
$R^4$ denotes methyl or ethyl,
$R^5$ denotes an alkyl group with 1 to 5 carbon atoms,
X denotes oxygen or sulfur and
Y denotes oxygen or sulfur, but X and Y cannot both be sulfur at the same time.

2. Pyrazolin-5-onyl-methyl-(di)thiophosphoric acid esters of the formula I according to claim 1, characterized in that $R^1$ and $R^2$ denote methyl, $R^3$ denotes hydrogen, $R^4$ denotes methyl or ethyl and $R^5$ denotes alkyl with 1 to 4 carbon atoms.

3. Process for the preparation of phosphoric acid esters of the formula I according to claim 1, characterized in that pyrazolinones of the formula II

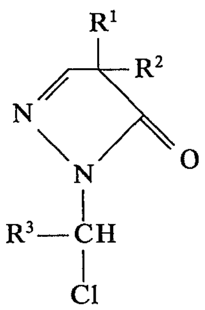

(II)

where $R^1$, $R^2$ and $R^3$ have the meanings given in claim 1, are reacted with (di)thiophosphoric acids of the formula III

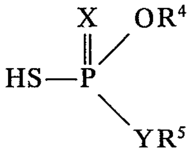

(III)

where $R^4$, $R^5$, X and Y have the meanings given in claim 1, in the presence of an acid acceptor, or with salts of the (di)thiophosphoric acids of the formula III, if appropriate in the presence of an organic solvent or diluent, at a temperature in the range of from 20 to 100° C.

4. Process for pest control, characterized in that a pyrazolin-5-onyl-methyl-(di)thiophosphoric acid ester of the formula I according to claim 1 is allowed to act on the pests or on their habitats.

**Revendications pour les Etats contractants: BE, CH, DE, FR, GB, IT, LI, NL, SE**

1. Ester de l'acide pyrazolin-5-on-yl-méthyl-(di)thiophosphorique de formule I

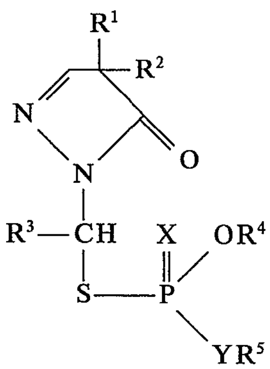

(I)

dans laquelle

$R^1$ et $R^2$ sont identiques ou différents et représentent un groupe alkyle à 1 à 5 atomes de carbone, allyle, phényle, $R^1$, en outre, méthoxycarbonylméthyle,

$R^3$ représente hydrogène ou un groupe méthyle éventuellement substitué par un halogène,

$R^4$ méthyle ou éthyle,

$R^5$ un groupe alkyle à 1 à 5 atomes de carbone,

X oxygène ou soufre et

Y oxygène ou soufre

X et Y ne pouvant être du soufre simultanément.

2. Ester d'acide pyrazolin-5-on-yl-méthyl-(di)thiophosphorique de formule I selon la revendication 1, caractérisé par le fait que $R^1$ et $R^2$ représentent méthyle, $R^3$ hydrogène, $R^4$ méthyle ou éthyle, et $R^5$ alkyle à 1 à 4 atomes de carbone.

3. Procédé de préparation d'esters d'acide phosphorique de formule I selon la revendication 1, caractérisé par le fait que l'on fait réagir, éventuellement en présence d'un solvant ou diluant organique, à une température de la zone comprise entre 20 et 100° C, la pyrazolinone de formule II

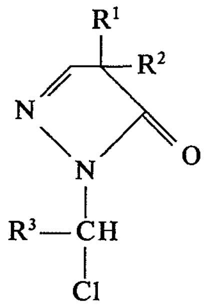

(II)

dans laquelle $R^1$, $R^2$, $R^3$ ont les significations données dans la revendication 1, avec des acides (di)thiophosphoriques de formule III

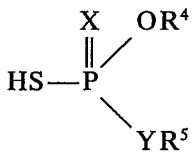

(III)

dans laquelle $R^4$, $R^5$, X et Y ont les significations données dans la revendication 1, en présence d'un agent liant d'acide ou avec des sels des acides (di)thiophosphoriques de formule III.

4. Agent de lutte contre les parasites, contenant un ester d'acide pyrazolin-5-on-yl-méthyl-(di)thiophosphorique de formule I selon la revendication 1.

5. Agent de lutte contre les parasites, contenant un support solide ou liquide et au moins un ester d'acide pyrazolin-5-on-yl-méthyl-(di)thiophosphorique de formule I selon la revendication 1.

6. Insecticide et acaricide contenant un ester d'acide pyrazolin-5-on-yl-méthyl-(di)thiophosphorique de formule I selon la revendication 1.

7. Procédé de lutte contre les parasites caractérisé par le fait qu'on fait réagir un ester d'acide pyrazolin-5-on-yl-méthyl-(di)thiophosphorique de formule I selon la revendication 1 sur les parasites ou leur biotope.

**Revendications pour l'Etat contractant: AT**

1. Agent de lutte contre les parasites, contenant un ester d'acide pyrazolin-5-on-yl-méthyl-(di)thiophosphorique de formule I

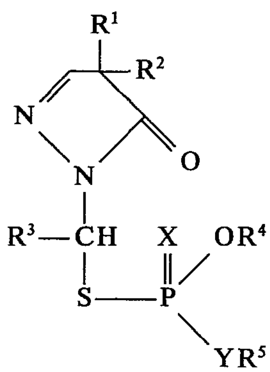

(I)

dans laquelle

$R^1$ et $R^2$ sont identiques ou différents et représentent un groupe alkyle à 1 à 5 atomes de carbone, allyle, phényle, $R^1$, en outre, méthoxycarbonylméthyle,
$R^3$ représente hydrogène ou un groupe méthyle éventuellement substitué par un halogène,
$R^4$ méthyle ou éthyle,
$R^5$ un groupe alkyle à 1 à 5 atomes de carbone,
X oxygène ou soufre et
Y oxygène ou soufre,
X et Y ne pouvant être du soufre simultanément.

2. Agent de lutte contre les parasites contenant un ester d'acide pyrazolin-5-on-yl-méthyl-(di)thiophosphorique de formule I selon la revendication 1, caractérisé par le fait que $R^1$ et $R^2$ représentent méthyle, $R^3$ hydrogène, $R^4$ méthyle ou éthyle, et $R^5$ alkyle à 1 à 4 atomes de carbone.

3. Procédé de préparation d'esters d'acide phosphorique de formule I selon la revendication 1, caractérisé par le fait que l'on fait réagir, éventuellement en présence d'un solvant ou diluant organique, à une température de la zone comprise entre 20 et 100° C, la pyrazolinone de formule II

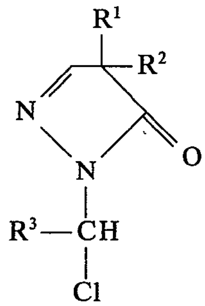

(II)

dans laquelle $R^1$, $R^2$, $R^3$ ont les significations données dans la revendication 1, avec des acides (di)thiophosphoriques de formule III

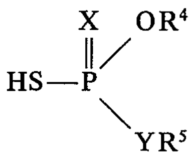

(III)

dans laquelle $R^4$, $R^5$, X et Y ont les significations données dans la revendication 1, en présence d'un agent liant d'acide ou avec des sels des acides (di)thiophosphoriques de formule III.

4. Procédé de lutte contre les parasites caractérisé par le fait qu'on fait réagir un ester d'acide pyrazolin-5-on-yl-méthyl-(di)thiophosphorique de formule I selon la revendication 1 sur les parasites ou leur biotope.